# EUROPEAN PATENT SPECIFICATION

(11) **EP 1 903 108 B1**
(45) Date of publication and mention of the grant of the patent: **02.12.2009**
(21) Application number: 07005065.3
(22) Date of filing: 12.03.2007
(51) Int. Cl.: C12N 9/02, C12N 15/00

(54) **Transmembrane domain-removed recombinant human tyrosinase and method of producing the same**
Rekombinante humane Tyrosinase mit beseitigter Transmembrandomäne und Herstellungsverfahren dafür
Tyrosinase humaine recombinante à domaine transmembranaire supprimé et son procédé de fabrication

(30) Priority: 20.09.2006 KR 20060091308
(43) Date of publication of application: 26.03.2008
(73) Proprietor: Chung-Ang University Industry-Academy Cooperation Foundation, Dongjak-gu Seoul 156-862 (KR)
(72) Inventor: Kong, Kwang-Hoon, Incheon 407-050 (KR)
(74) Representative: Bockhorni & Kollegen

(56) References cited:
- DATABASE WPI Week 200466 Derwent Publications Ltd., London, GB; AN 2004-673910 XP002430912 & KR 2004 045 961 A (KONG K H) 5 June 2004 (2004-06-05)
- KONG K H ET AL: "Expression and characterization of human tyrosinase from a bacterial expression system." COMPARATIVE BIOCHEMISTRY AND PHYSIOLOGY. PART B, BIOCHEMISTRY & MOLECULAR BIOLOGY APR 2000, vol. 125, no. 4, April 2000 (2000-04), pages 563-569, XP002430796 ISSN: 1096-4959
- DATABASE WPI Week 200377 Derwent Publications Ltd., London, GB; AN 2003-826022 XP002430913 & KR 2003 023 423 A (CHOI S S) 19 March 2003 (2003-03-19)
- KWON B S ET AL: "ISOLATION AND SEQUENCE OF A CDNA CLONE FOR HUMAN TYROSINASE THAT MAPS AT THE MOUSE C-ALBINO LOCUS" PROCEEDINGS OF THE NATIONAL ACADEMY OF SCIENCES OF USA, NATIONAL ACADEMY OF SCIENCE, WASHINGTON, DC, US, vol. 84, no. 21, 1987, pages 7473-7477, XP000887382 ISSN: 0027-8424 -& DATABASE UniProt [Online] 1 April 1990 (1990-04-01), "Tyrosinase precursor (EC 1.14.18.1) (Monophenol monooxygenase) (Tumor rejection antigen AB) (SK29-AB) (LB24-AB)." XP002430816 retrieved from EBI accession no. UNIPROT:P14679 Database accession no. P14679 -& DATABASE EMBL [Online] 24 April 1990 (1990-04-24), "Human tyrosinase (TYR) mRNA, complete cds." XP002430817 retrieved from EBI accession no. EMBL:M27160 Database accession no. M27160

### SEQUENCE LISTING

<110> Chung-Ang University Industry-Academy Coorperation Foundation
<120> Transmembrane Domain Removed Recombinant Human Tyrosinase And Method of Producing The Same
<150> KR 10-2006-0091308
   <151> 2006-09-20
<160> 4
<170> KopatentIn 1.71
<210> 1
   <211> 1371
   <212> DNA
   <213> Human tyrosinase
<220>
   <221> CDS
   <222> (1)..(1371)
<400> 1
   atg cac ttc cct aga gcc tgt gtc tcc tct aag aac ctg atg gag aag 48
   Met His Phe Pro Arg Ala Cys Val Ser Ser Lys Asn Leu Met Glu Lys
   1 5 10 15
   gaa tgc tgt cca ccg tgg agc ggg gac agg agt ccc tgt ggc cag ctt 96
   Glu Cys Cys Pro Pro Trp Ser Gly Asp Arg Ser Pro Cys Gly Gln Leu
   20 25 30
   tca ggc aga ggt tcc tgt cag aat atc ctt ctg tcc aat gca cca ctt       144
   Ser Gly Arg Gly Ser Cys Gln Asn Ile Leu Leu Ser Asn Ala Pro Leu
           35                  40                  45
   ggg cct caa ttt ccc ttc aca ggg gtg gat gac cgg gag tcg tgg cct       192
   Gly Pro Gln Phe Pro Phe Thr Gly Val Asp Asp Arg Glu Ser Trp Pro
       50                  55                  60
   tcc gtc ttt tat aat agg acc tgc cag tgc tct ggc aac ttc atg gga       240
   Ser Val Phe Tyr Asn Arg Thr Cys Gln Cys Ser Gly Asn Phe Met Gly
   65                  70                  75                  80
   ttc aac tgt gga aac tgc aag ttt ggc ttt tgg gga cca aac tgc aca       288
   Phe Asn Cys Gly Asn Cys Lys Phe Gly Phe Trp Gly Pro Asn Cys Thr
                   85                  90                  95
   gag aga cga ctc ttg gtg aga aga aac atc ttc gat ttg agt gcc cca       336
   Glu Arg Arg Leu Leu Val Arg Arg Asn Ile Phe Asp Leu Ser Ala Pro
               100                 105                 110
   gag aag gac aaa ttt ttt gcc tac ctc act tta gca aag cat acc atc       384
   Glu Lys Asp Lys Phe Phe Ala Tyr Leu Thr Leu Ala Lys His Thr Ile
           115                 120                 125
   agc tca gac tat gtc atc ccc ata ggg acc tat ggc caa atg aaa aat       432
   Ser Ser Asp Tyr Val Ile Pro Ile Gly Thr Tyr Gly Gln Met Lys Asn
       130                 135                 140
   gga tca aca ccc atg ttt aac gac atc aat att tat gac ctc ttt gtc       480
   Gly Ser Thr Pro Met Phe Asn Asp Ile Asn Ile Tyr Asp Leu Phe Val
   145                 150                 155                 160
   tgg atg cat tat tat gtg tca atg gat gca ctg ctt ggg gga tct gaa       528
   Trp Met His Tyr Tyr Val Ser Met Asp Ala Leu Leu Gly Gly Ser Glu
                   165                 170                 175
   atc tgg aga gac att gat ttt gcc cat gaa gca cca gct ttt ctg cct       576
   Ile Trp Arg Asp Ile Asp Phe Ala His Glu Ala Pro Ala Phe Leu Pro
               180                 185                 190
   tgg cat aga ctc ttc ttg ttg cgg tgg gaa caa gaa atc cag aag ctg       624
   Trp His Arg Leu Phe Leu Leu Arg Trp Glu Gln Glu Ile Gln Lys Leu
           195                 200                 205
   aca gga gat gaa aac ttc act att cca tat tgg gac tgg cgg gat gca       672
   Thr Gly Asp Glu Asn Phe Thr Ile Pro Tyr Trp Asp Trp Arg Asp Ala
       210                 215                 220
   gaa aag tgt gac att tgc aca gat gag tac atg gga ggt cag cac ccc       720
   Glu Lys Cys Asp Ile Cys Thr Asp Glu Tyr Met Gly Gly Gln His Pro
   225                 230                 235                 240
   aca aat cct aac tta ctc agc cca gca tca ttc ttc tcc tct tgg cag       768
   Thr Asn Pro Asn Leu Leu Ser Pro Ala Ser Phe Phe Ser Ser Trp Gln
                   245                 250                 255
   att gtc tgt agc cga ttg gag gag tac aac agc cat cag tct tta tgc       816
   Ile Val Cys Ser Arg Leu Glu Glu Tyr Asn Ser His Gln Ser Leu Cys
               260                 265                 270
   aat gga acg ccc gag gga cct tta cgg cgt aat cct gga aac cat gac       864
   Asn Gly Thr Pro Glu Gly Pro Leu Arg Arg Asn Pro Gly Asn His Asp
           275                 280                 285
   aaa tcc aga acc cca agg ctc ccc tct tca gct gat gta gaa ttt tgc      912
   Lys Ser Arg Thr Pro Arg Leu Pro Ser Ser Ala Asp Val Glu Phe Cys
       290                 295                 300
   ctg agt ttg acc caa tat gaa tct ggt tcc atg gat aaa gct gcc aat      960
   Leu Ser Leu Thr Gln Tyr Glu Ser Gly Ser Met Asp Lys Ala Ala Asn
   305                 310                 315                 320
   ttc agc ttt aga aat aca ctg gaa gga ttt gct agt cca ctt act ggg     1008
   Phe Ser Phe Arg Asn Thr Leu Glu Gly Phe Ala Ser Pro Leu Thr Gly
                   325                 330                 335
   ata gcg gat gcc tct caa agc agc atg cac aat gcc ttg cac atc tat     1056
   Ile Ala Asp Ala Ser Gln Ser Ser Met His Asn Ala Leu His Ile Tyr
               340                 345                 350
   atg aat gga aca atg tcc cag gta cag gga tct gcc aac gat cct atc     1104
   Met Asn Gly Thr Met Ser Gln Val Gln Gly Ser Ala Asn Asp Pro Ile
           355                 360                 365
   ttc ctt ctt cac cat gca ttt gtt gac agt att ttt gag cag tgg ctc     1152
   Phe Leu Leu His His Ala Phe Val Asp Ser Ile Phe Glu Gln Trp Leu
       370                 375                 380
   cga agg cac cgt cct ctt caa gaa gtt tat cca gaa gcc aat gca ccc     1200
   Arg Arg His Arg Pro Leu Gln Glu Val Tyr Pro Glu Ala Asn Ala Pro
   385                 390                 395                 400
   att gga cat aac cgg gaa tcc tac atg gtt cct ttt ata cca ctg tac     1248
   Ile Gly His Asn Arg Glu Ser Tyr Met Val Pro Phe Ile Pro Leu Tyr
                   405                 410                 415
   aga aat ggt gat ttc ttt att tca tcc aaa gat ctg ggc tat gac tat        1296
   Arg Asn Gly Asp Phe Phe Ile Ser Ser Lys Asp Leu Gly Tyr Asp Tyr
               420                 425                 430
   agc tat cta caa gat tca gac cca gac tct ttt caa gac tac att aag        1344
   Ser Tyr Leu Gln Asp Ser Asp Pro Asp Ser Phe Gln Asp Tyr Ile Lys
           435                 440                 445
   tcc tat ttg gaa caa gcg agt cgg atc                                    1371
   Ser Tyr Leu Glu Gln Ala Ser Arg Ile
       450                 455
<210> 2
   <211> 457
   <212> PRT
   <213> Human tyrosinase
<400> 2
   Met His Phe Pro Arg Ala Cys Val Ser Ser Lys Asn Leu Met Glu Lys
   1               5                   10                  15
   Glu Cys Cys Pro Pro Trp Ser Gly Asp Arg Ser Pro Cys Gly Gln Leu
               20                  25                  30
   Ser Gly Arg Gly Ser Cys Gln Asn Ile Leu Leu Ser Asn Ala Pro Leu
           35                  40                  45
   Gly Pro Gln Phe Pro Phe Thr Gly Val Asp Asp Arg Glu Ser Trp Pro
       50                  55                  60
   Ser Val Phe Tyr Asn Arg Thr Cys Gln Cys Ser Gly Asn Phe Met Gly
   65 70 75 80
   Phe Asn Cys Gly Asn Cys Lys Phe Gly Phe Trp Gly Pro Asn Cys Thr
   85 90 95
   Glu Arg Arg Leu Leu Val Arg Arg Asn Ile Phe Asp Leu Ser Ala Pro
   100 105 110
   Glu Lys Asp Lys Phe Phe Ala Tyr Leu Thr Leu Ala Lys His Thr Ile
   115 120 125
   Ser Ser Asp Tyr Val Ile Pro Ile Gly Thr Tyr Gly Gln Met Lys Asn
   130 135 140
   Gly Ser Thr Pro Met Phe Asn Asp Ile Asn Ile Tyr Asp Leu Phe Val
   145 150 155 160
   Trp Met His Tyr Tyr Val Ser Met Asp Ala Leu Leu Gly Gly Ser Glu
   165 170 175
   Ile Trp Arg Asp Ile Asp Phe Ala His Glu Ala Pro Ala Phe Leu Pro
   180 185 190
   Trp His Arg Leu Phe Leu Leu Arg Trp Glu Gln Glu Ile Gln Lys Leu
   195 200 205
   Thr Gly Asp Glu Asn Phe Thr Ile Pro Tyr Trp Asp Trp Arg Asp Ala
   210 215 220
   Glu Lys Cys Asp Ile Cys Thr Asp Glu Tyr Met Gly Gly Gln His Pro
   225 230 235 240
   Thr Asn Pro Asn Leu Leu Ser Pro Ala Ser Phe Phe Ser Ser Trp Gln
   245 250 255
   Ile Val Cys Ser Arg Leu Glu Glu Tyr Asn Ser His Gln Ser Leu Cys
   260 265 270
   Asn Gly Thr Pro Glu Gly Pro Leu Arg Arg Asn Pro Gly Asn His Asp
   275 280 285
   Lys Ser Arg Thr Pro Arg Leu Pro Ser Ser Ala Asp Val Glu Phe Cys
   290 295 300
   Leu Ser Leu Thr Gln Tyr Glu Ser Gly Ser Met Asp Lys Ala Ala Asn
   305 310 315 320
   Phe Ser Phe Arg Asn Thr Leu Glu Gly Phe Ala Ser Pro Leu Thr Gly
   325 330 335
   Ile Ala Asp Ala Ser Gln Ser Ser Met His Asn Ala Leu His Ile Tyr
   340 345 350
   Met Asn Gly Thr Met Ser Gln Val Gln Gly Ser Ala Asn Asp Pro Ile
   355 360 365
   Phe Leu Leu His His Ala Phe Val Asp Ser Ile Phe Glu Gln Trp Leu
   370 375 380
   Arg Arg His Arg Pro Leu Gln Glu Val Tyr Pro Glu Ala Asn Ala Pro
   385 390 395 400
   Ile Gly His Asn Arg Glu Ser Tyr Met Val Pro Phe Ile Pro Leu Tyr
   405 410 415
   Arg Asn Gly Asp Phe Phe Ile Ser Ser Lys Asp Leu Gly Tyr Asp Tyr
   420 425 430
   Ser Tyr Leu Gln Asp Ser Asp Pro Asp Ser Phe Gln Asp Tyr Ile Lys
   435 440 445
   Ser Tyr Leu Glu Gln Ala Ser Arg Ile
   450 455
<210> 3
   <211> 40
   <212> DNA
   <213> Artificial Sequence
<220>
   <223> forward primer
<400> 3
   ggaattccat atgcacttcc ctagagcctg tgtctcctct 40
<210> 4
   <211> 31
   <212> DNA
   <213> Artificial Sequence
<220>
   <223> reverse primer
<400> 4
   ccgctcgagc gggatccgac tcgcttgttc c 31

## Description

This application claims the priority of Korea Patent application No. 2006-0091308 filed on September 20, 2006.

### FIELD OFTHE INVENTION

The present invention relates to a recombinant human tyrosinase, which transmembrane domain of human tyrosinase is removed and a method of producing the same, and more particularly to a recombinant human tyrosinase and a method of producing the same, in which the recombinant human tyrosinase is produced through expressing recombinant human tyrosinase gene, that the carboxylic terminal transmembrane domain of human tyrosinase is removed, and then separating and purifying the expressed recombinant human tyrosinase.

### BACKGROUND OF THE INVENTION

The formation and surface deposition of melanin dye at cutis is natural phenomenon to protect a body from harmful ultraviolet of sunlight. This phenomenon is a kind of a body protecting system. High polymeric melanin dye present at the cutis absorbs the ultraviolet to transform it into thermal energy to disperse. In addition, the melanin dye absorbs free radicals produced through the reaction of the ultraviolet with lipid of the cutis and other molecules inducing the oxidative damage to the cutis cell, thereby protecting the body. When the cutis deficient of the melanin dye genetically is exposed to the ultraviolet, the thickness of cutis horny layer becomes thick. The protein included in the horny layer scatters or disperses the ultraviolet to protect the body. Generally, when the cutis is exposed to ultraviolet, melanocytes present under the cutis instantly produces melanin. Thus produced melanin is transferred to keratinocytes and is carried to the upper portion of the epidermis so that the cutis forms an ultraviolet protection layer (SPF: sun protection factor of the layer is about 3-5) of which color is changed to black. However, when the produced melanin dye is dispersed non-uniformly while being abnormally combined together, freckles, ephelis, etc. are formed. When the melanin is formed at the melanocytes, tyrosinase starts to function. At initial stage of melanin production, the tyrosinase catalyzes tyrosine (hydroxy phenyl alanine), which is one of the amino acids, to dihydroxy phenyl alanine (DOPA). After that, the tyrosinase promotes the oxidation of dihydroxy phenyl alanine (DOPA) to transform into quinone (DOPA quinone). Thus formed DOPA quinone is passed through several steps, thereby to be catalyzed to melanin.

Tyrosinase (monophenol monooxygenase, EC 1.14.18.1) is widely spread in mammal, plants, etc. Phyto-tyrosinase is concerned with the browning during processing fruits and vegetables. The tyrosinase of the mammal is included in a melanocyte dispersed in cutis, hair follicle, eye and the like and functions to prevent the damage due to ultraviolet. Until now, various kinds of tyrosinase have been isolated and purified from various living organisms. Among them, researches on the structure and function of tyrosinase isolated from a mushroom and a mouse are most actively implemented (Laskin and Paccinini, J. Biol. Chem. 261: 16626-16635, 1986; Mayer, Phytochem. 26: 11-20, 1987; Jimenez-Cervantes et al., Pigment Cell Res. 6: 394-399, 1993; Sanchez-Ferrer et al., Biochim Biophys Acta. 1247: 1-5, 1995; Della Longa et al., J. Biol. Chem. 35: 21025-21030, 1996). Tyrosinase is a metalloprotein including a pair of copper ions at the active site. The human tyrosinase has a molecular weight of about 66,000 Da The tyrosinase except hydrophobic signal peptide consists of 548 amino acids and has a molecular weight of 62,150 Da. Tyrosinase has tyrosine hydroxylase activity of catalyzing a hydroxylation reaction to transform monohydroxyphenol into O-dihydroxyphenol as shown in the following reaction mechanism 1. Tyrosinase also has Dopa oxidase activity of catalyzing an oxidation reaction to transform O-dihydroxyphenol into O-quinone (Mason, Annu Rev. Biochem. 34: 105-184, 1965).

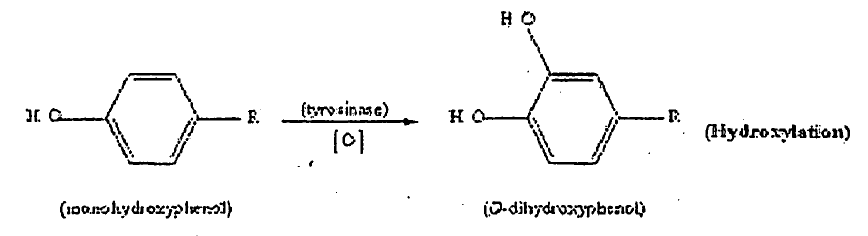

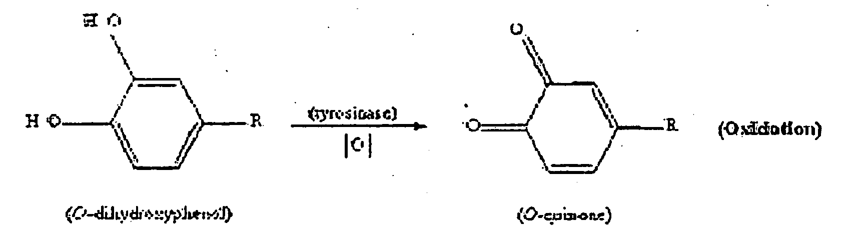

Human tyrosinase is isolated from melanoma cell (Wittbjer, Acta Derm. Venereol. 69: 125-131, 1989; Wittbjer, Acta Derm. Venereol. 70: 291-294, 1990). In U.S. Patent No. 4,898,814 issued in 1990, human tyrosinase cDNA and its nucleotide sequence is disclosed. PCT Patent Application Publication No. WO 90/12869 discloses a method of producing active human tyrosinase using non-melanocytic eukaryotic cell. The method includes inserting DNA encoding the human tyrosinase into an appropriate expression vector such as a retrovirus to produce a novel transformation vector and then introducing the vector into eukaryotic cell such as fibroblast. Thus transformed cell is cultured, separated and purified to obtain the human tyrosinase. However, according to the above described method, the DNA encoding human tyrosinase is inserted into the expression vector such as the retrovirus and then transformed into the fibroblast. Then, the fibroblast is cultured to increase the amount of the tyrosinase and then homogenized. The human tyrosinase is separated and purified from cytoplasm in which various cell organelles are present. Therefore, the method is technically complicated and difficult and has disadvantages of difficulties of commercialization through a mass production. Meanwhile, the present inventors have filed a method of producing recombinant human tyrosinase using *E. coli* (Korean Patent Application Nos. 2001-0057379 and 2002-0073728) to the Korean Intellectual Property Office as the previous research. However, according to the above-described method, there was a disadvantage of reducing the purification yield due to the two step purification using DEAE-sephacel ion exchange chromatography and His-bind resin chromatography.

### SUMMARY OF THE INVENTION

Considering the above disadvantages, the present inventors continued a research on the method of producing recombinant human tyrosinase and found that when the recombinant human tyrosinase gene, that carboxy-terminal transmembrane domain is removed from human tyrosinase gene, is expressed, the recombinant human tyrosinase having a high enzyme activity can be produced without forming insoluble inclusion body.

The purification yield can be improved only when applying the first purification using the His·bind resin chromatography.

Accordingly, an object of the present invention is to provide a recombinant human tyrosinase and a method of producing the same.

### BRIEF DESCRIPTION OF THE DRAWINGS

The above and other objects, features and advantages of the present invention will be more apparent from the following detailed description taken in conjunction with the accompanying drawings, in which:
FIG. 1 is a diagram for preparing process of recombinant expression vector pET-hTyrosinase(TM-) according to the present invention; and
FIG. 2 is a result of 12.5% SDS-PAGE to confirm the recombinant human tyrosinase purified from a crude extract, in which lane M represents molecular weight marker, lane 1 represents crude extract and lane 2 represents the recombinant human tyrosinase of the present invention purified by His·bind resin chromatography.

### DETAILED DESCRIPTION OF THE INVENTION

To accomplish the above object, the present invention provides a human tyrosinase consisting of the amino acid sequence set forth in SEQ ID NO: 2.

The present invention also provides a polynucleotide encoding the human tyrosinase.

The present invention further provides a recombinant vector comprising the polynucleotide and bacteria transformed with the vector.

The present invention further provides a method of producing the human tyrosinase comprising the steps of culturing the transformed *E*. *coli* and purifying the protein.

Hereinafter, the present invention will be described in more detail.

The present invention provides a recombinant human tyrosinase.

In more particularly, the present invention provides a human tyrosinase consisting of the amino add sequence set forth in SEQ ID NO: 2.

Enzymatic characteristic of the recombinant human tyrosinase is summarized as follows.
1) molecular weight: 53kDa
2) specific activity
   specific activity to tyrosine hydroxylase activity: 108.3 ± 2.10 µmol/mg/h
   specific activity to Dopa oxidase activity: 807 units/mg
3) Km value to a substrate
   L-tyrosine: 1.32 µM
   L-Dopa: 0.34 mM

The recombinant human tyrosinase of the present invention is a protein, that transmembrane domain is removed from a human tyrosinase amino acid. The full-length nucleotide sequence of the human tyrosinase is described in GenBank Accession No. M27160. The protein of the present invention is encoded by the nucleotide of 557 to the nucleotide of 1927, which is except for the N-terminal signal sequence and the C-terminal transmembrane domain.

As used herein, the term "the removal of the transmembrane domain" from the human tyrosinase sequence means the removal of 55 amino acids from the carboxyl terminal of the human tyrosinase. As used herein, the term "the recombinant human tyrosinase of the present invention" means a recombinant polypeptide produced by the removal of the transmembrane domain, that is, the transmembrane domain of human tyrosinase is removed from human tyrosinase to form the recombinant tyrosinase of the present invention.

The recombinant human tyrosinase having the above-mentioned characteristic according to the present invention may be a polypeptide consisting of the amino acid sequence set forth in SEQ ID NO: 2. The recombinant human tyrosinase of the present invention includes the functional equivalents of the polypeptide.

As used herein, the term "functional equivalents" refers to polypeptides having a sequence homology (i.e., identity) of at least 70%, preferably at least 80%, and more preferably at least 90% to the amino acid sequence set forth in SEQ ID NO:2 as results of addition, substitution and deletion of amino acid which exhibit substantially identical physiological activity to the recombinant human tyrosinase consisting of an amino acid sequence set forth in SEQ ID NO: 2.

The term "substantially identical physiological activity" refers to tyrosine hydroxylase activity and dopa oxidase activity. The functional equivalents may be polypeptides having a sequence homology (i.e., identity) of at least 70%, preferably at least 80%, and more preferably at least 90% to the amino acid sequence set forth in SEQ ID NO:2. For example, the term functional equivalents refers to polypeptides comprising the amino acid sequence having sequence homology of 70%, 71%, 72%, 73%, 74%, 75%, 76%, 77%, 78%, 79%, 80%, 81%, 82%, 83%, 84%, 85%, 86%, 87 %, 88 %, 89%, 90%, 91%, 92%, 93%, 94%, 95%, 96%, 97%, 98%, 99% and 100% to the amino acid sequence of SEQ ID NO: 2. The functional equivalents may include, for example peptides produced by as a result of addition, substitution or deletion of some amino acid of SEQ ID NO: 2. Substitutions of the amino acids are preferably conservative substitutions. Examples of conservative substitutions of naturally occurring amino acids are as follows: aliphatic amino acids (Gly, Ala, Pro), hydrophobic amino acids (Ile, Leu, Val), aromatic amino acids (Phe, Tyr, Trp), acidic amino acids (Asp, Glu), basic amino acids (His, Lys, Arg, GIn, Asn) and sulfur-containing amino acids (Cys, Met). Furthermore, the functional equivalents also include variants with deletion of some of the amino acid sequence of the recombinant human tyrosinase of the present invention. The deletion or substitutions of the amino acids are preferably located at regions that are not directly involved in the physiological activity of the recombinant human tyrosinase of the present invention. In addition, the functional equivalents also include variants with addition of several amino acids in both terminal ends of the amino acid sequence of the recombinant human tyrosinase of the present invention or in the sequence. Moreover, the functional equivalents also include polypeptide derivatives which have modification of some of the chemical structure of the recombinant human tyrosinase of the present invention while maintaining the fundamental backbone and physiological activity of the recombinant human tyrosinase of the present invention. Examples of this modification include structural modifications for changing the stability, storage, volatility or solubility of the recombinant human tyrosinase of the present invention.

Sequence identity or homology is defined herein as the percentage of amino acid residues in the candidate sequence that are identical with amino acid sequence of the recombinant human tyrosinase of the present invention (SEQ ID NO: 2), after aligning the sequences and introducing gaps, if necessary, to achieve the maximum percent sequence identity, and not considering any conservative substitutions (as described above) as part of the sequence identity. None of N-terminal, C-tenninal, or internal extensions, deletions, or insertions into the amino acid sequence of the recombinant human tyrosinase of the present invention shall be construed as affecting sequence identity or homology. Thus, sequence identity can be determined by standard methods that are commonly used to compare the similarity in position of the amino acids of two polypeptides. Using a computer program such as BLAST or FASTA, two polypeptides are aligned for optimal matching of their respective amino acids (either along the full length of one or both sequences or along a predetermined portion of one or both sequences). The programs provide a default opening penalty and a default gap penalty, and a scoring matrix such as PAM 250 (a standard scoring matrix; see Dayhoff et al., in Atlas of Protein Sequence and Structure, vol. 5, supp. 3 (1978)) can be used in conjunction with the computer program. For example, the percent identity can be calculated as: the total number of identical matches multiplied by 100 and then divided by the sum of the length of the longer sequence within the matched span and the number of gaps introduced into the longer sequences in order to align the two sequences.

Also, the present invention provides polynucleotide encoding the recombinant human tyrosinase of the present invention. Preferably, the polynucleotide may be DNA or RNA consisting of the nucleotide sequence set forth in SEQ ID NO: 1. The polynucleotide can be isolated from the nature or can be prepared by a chemical synthesis.

The present invention also provides a recombinant vector comprising the polynucleotide encoding the recombinant human tyrosinase of the present invention.

In the present invention, "recombinant vector" is a genetic carrier to express a protein or RNA of interest in a suitable host cell, comprising a corresponding foreign sequence operably linked to a nucleic acid expression control sequence (such as a promoter, signal sequence and array of transcription factor binding sites).

The term "operably linked" used herein refers to functional linkage between a nucleic acid expression control sequence and a second nucleic acid sequence of interest, wherein the expression control sequence affects transcription and/or translation of the nucleic acid corresponding to the second sequence. The vector of this invention may be constructed according to conventional recombinant DNA technology in which site-specific DNA cleavage and ligation are performed using commercially available enzymes known in the art.

The vector of the present invention includes plasmid, cosmid, bacteriophage and viral vectors, but not limited to. The vector may comprise expression control elements such as promoter, operator, start and stop codons, polyadenylation signal and enhancer as well as signal or leader sequences for membrane targeting or secretion. The promoter used in vectors may be constitutive or inducible one. Furthermore, the vector may carry a selection marker for selecting host cells harboring the vector and a replication origin. The signal sequence in the vector includes, but not limited to, PhoA and OmpA signal sequences for *Escherichia* host cells, alpha-amylase and subtilicin signal sequences for Bacillus host cells, MF alpha and SUC2 signal sequences for yeast host cells, and insulin, alpha-interferon and antibody molecule signal sequences for animal host cells.

In one embodiment of the present invention, for the purpose of easy protein purification, pET26B(+) vector (Novagen) having His Tag sequence is used in order to express the recombinant human tyrosinase which artificially includes six histidines.

The present invention also provides a transformant which is transformed with the recombinant vector of the present invention. The transformation may be carried out according to any known methods for introducing nucleic acid molecules into organism, cell, tissue or organ. A suitable transformation method may be selected based on the type of host cells. Examples of these methods include electroporation, protoplasm fusion, CaPO₄ precipitation, CaCl₂ precipitation, agitation using silicon carbamide fiber, Agrobacterium-mediated transformation, PEG, dextran sulfate and lipofectamine, but not limited thereto.

A suitable host cell is generally decided in considering the expression level and post-translational modification. Examples of host cells include, but not limited to, prokaryotic cells such as *Escherichia coli, Bacillus subtilis, Streptomyces, Pseudomonas, Proteus mirabilis* and *Staphylococcus.* Lower eukaryotes such as fungi (e.g., *Aspergillus),* yeast (e.g., *Pichia pastoris, Saccharomyces cerevisiae, Schizosaccharomyces* and *Neurospora crassa* and the like) and higher eukaryotes such as insect cells, plant cells and mammalian cells and the like may be used as host cell. Preferably, the host cell is *E. coli.*

Also, present invention provides a method of producing a recombinant human tyrosinase comprising steps of culturing a transformant transformed with a recombinant vector having a gene encoding the recombinant human tyrosinase of the present invention and purifying the tyrosinase.

The method comprises the step of culturing the transformed host cell so that polynucleotide encoding the recombinant human tyrosinase of the present invention can be expressed under an appropriate medium and condition. The method of culturing the transformed cell and expressing the recombinant protein is known in the art. For example, the transformed cell is inoculated in an appropriate culture medium for a seed culture. After that, thus obtained product is inoculated onto a culture medium for a main culturing and is cultured under an appropriate condition to induce a protein expression. The isolation and purification of the recombinant human tyrosinase of the present invention induced and expressed from the transformed cell can be implemented by means of various separation and purification methods known in the art. For example, the cell is suspended and centrifuged. Then, a salting out (e.g. ammonium sulfate precipitation and sodium phosphate precipitation), a solvent precipitation (e.g. protein fractionation precipitation using acetone, ethanol, etc.), dialysis, gel filtration, ion exchange chromatography, reverse-phase column chromatography, affinitive chromatography, etc. can be applied alone or in combination to produce the recombinant human tyrosinase of the present invention (Maniatis et al., Molecular Cloning: A Laboratory Manual, Cold Spring Harbor Laboratory, Cold Spring Harbor, N.Y.(1982); Sambrook et al., Molecular Cloning: A Laboratory Manual, 2nd Ed., Cold Spring Harbor Laboratory Press(1989); Deutscher, M., Guide to Protein Purification Methods Enzymology, vol. 182. Academic Press. Inc., San Diego, CA(1990)).

The recombinant human tyrosinase can be produced through inducing the expression of the recombinant human tyrosinase of the present invention by culturing *E*. *coli* introducing an expression vector pET-hTyrosinase(TM-), disrupting the *E. coli,* centrifuging to obtain the supernatant, and purifying by means of His bind resin chromatography (Novagen).

As described above, the method of producing the recombinant human tyrosinase of the present invention has a characteristic that an additional purifying process using DEAE-sephacel ion exchange chromatography is not required. In order to use His bind resin chromatography, the expression of recombinant protein to which six histidines is artificially included is needed. In the case that the six histidines are partially covered due to the inherent structure of the protein, imidazole having a low mole number should be applied in purification using His-bind resin chromatography. In order to purify the recombinant human tyrosinase produced in *E*. *coli* using pET-hTyr expression vector produced according to a method described in the previous research by the present inventors in comparative example 2 (Korean Patent Application No. 2002-0073728), imidazole having a low mole number should be used since the six histidines artificially included at the C-terminal of the tyrosinase are covered structurally. The utilization of imidazole having low mole number leads to the elucidation of other unnecessary protein other than the recombinant human tyrosinase. Therefore, a purifying process using the DEAE-sephacel ion exchange chromatography is needed before the application of the His-bind resin chromatography. However, for the recombinant human tyrosinase of the present invention, the structural covering problem of six histidines can be solved and imidazole having high mole number can be used. Therefore, the purification without the DEAE-sephacel ion exchange chromatography, can be possibly carried out.

In addition, with the method of producing the recombinant human tyrosinase of the present invention, an insoluble inclusion body is not formed. Therefore, the present invention has a characteristic that a surfactant is not required during the producing steps. The transmembrane domain is generally composed of hydrophobic amino acid. The hydrophobic residues of amino acid are liable to combine each other within an aqueous solution. When the surfactant is used, the formation of insoluble inclusion body due to the transmembrane domain composed of the hydrophobic residue can be prevented to some degree. In the previously application by the present inventors (Korean patent Application No. 2002-0073728), 1% triton X-100 was used as the surfactant to prevent the formation of the insoluble inclusion body of the tyrosinase protein. However, in the present invention, the transmembrane domain of the tyrosinase is removed to prevent the formation of the insoluble inclusion body without using 1% triton X-100, thereby producing the human tyrosinase having a high enzyme activity.

The method according to the present invention has a characteristic that an additional step of removing histidine tag during purifying protein is not necessary. In order to facilitate the purification of the protein, the recombinant human tyrosinase of the present invention is expressed in the form that six histidines are artificially added. According to the method disclosed in the previous application by the present inventors (Korean Patent Application No. 2001-0057379), a removing step of histidine is required because histidines positioned at N-terminal affect the activity of the tyrosinase. However, in the present invention, a vector system is constructed so that the histidine is positioned at C-tenninal position not to affect the activity of the histidine. Therefore, the removing step of histidine is not necessary.

### BEST MODE FOR CARRYING OUT THE INVENTION

Hereinafter, the present invention will be described in more detail referring to the embodiments. However, the following embodiments are illustrative purposes only and the content of the present invention is not limited to the following examples.

### <Example 1 >

### Cloning and expression of the recombinant human tyrosinase gene

### <1-1> preparation of primer

Primers were designed based on cDNA sequence encoding tyrosinase of melanoma DNA disclosed in GenBank accession No. M27160. The primers were designed so that the nucleotides except N-terminal signal sequence and C-terminal transmembrane domain (from No. 55 nucleotide to No. 1927 nucleotide of M27160) were synthesized. To be translated to protein, in front of the No 557 nucleotide of M27160, ATG coding Met was artificially inserted into sense primer. To induce an advantageous synthesis of protein using *E*. *coli* codon usage, CAT sequence behind an initiation codon ATG is replaced with CAC and a restriction enzyme sites of *Nde* I and *Xho* I were added in the primer, respectively. The *Nde* I and *Xho* I site are underlined.
Sense primer (40 mer): 5'. GGAATTC**CATATG**CACTTCCCTAGAGCCTGTGTCTCCTCT-3' (SEQ ID NO : 3)
Anti-sense primer (31 mer): 5'-CCG**CTCGAG**CGGGATCCGACTCGCTTGTTCC-3' (SEQ ID NO : 4)

### <1-2> PCR amplification of the recombinant human tyrosinase gene

In order to cloning the human tyrosinase gene which encode the recombinant human tyrosinase of the present invention, PCR was conducted by using human 5'-stretch plus cDNA library(Clontech, Palo Alto CA, USA), as a template.

PCR was conducted by using a PCR reaction solution having a total volume of 50 µℓ and including 100 ng of template DNA, 0.8 µM of sense and anti-sense primer, 0.2 mM of a final concentration of dNTP mixture (TaKaRa), 5 µℓ of 10 X Z-Tag buffer solution and 1 µℓ of Z-Tag polymerase (TaKaRa). The PCR reaction was conducted by 35 cycles of 10 seconds at 980, 30 seconds at 65□, and 60 seconds at 720. After that, a 1% Agarose gel electrophoresis was conducted to confirm an amplified PCR product. DNA was extracted from the gel by means of gene clean kit(BIO 101).

### <1-3> Cloning and expression

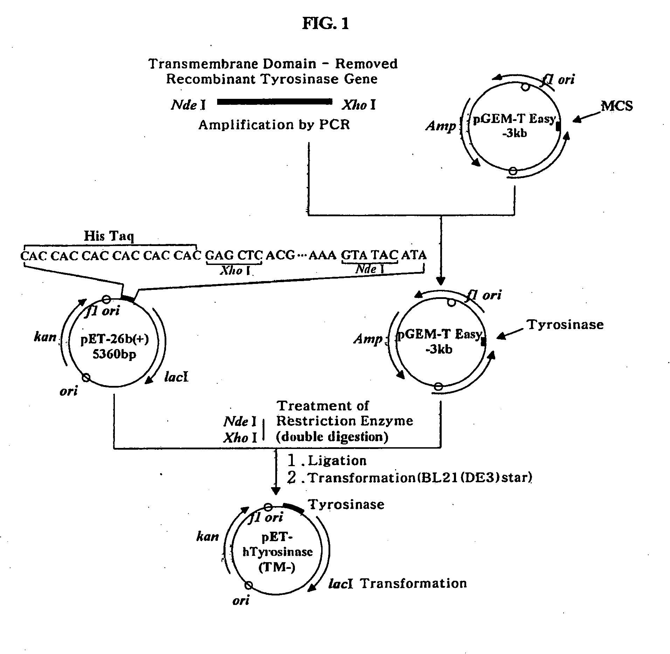

### Cloning process of the amplified PCR product is illustrated in FIG. 1.

First, the DNA fragment extracted from the gel and having a size of 1399 bp, was mixed with a ligation reacting solution (pGEM-T easy vector 0.3 µℓ, 2 x buffer 2.5 µℓ, ligase 0.5 µℓ) and then thus obtained product was inserted into pGEM-T Easy vector (Promega. USA). After that, *E*. *coli* BL21 star (DE3) was transformed with pGEM-T Easy vector to which the tyrosinase gene was inserted by heat shock for 40 seconds at 42□. After the transformation, the transformed *E. coli* was selected by culturing L-Broth plate having 50µg/mℓ Ampicillin. A colony was selected and cultured on a liquid L-Broth for DNA extraction.

Meanwhile, six histidines were added to the recombinant human tyrosinase to facilitate the purification of the recombinant human tyrosinase of the present invention. For this, the human tyrosinase gene was inserted into pET26B(+) vector (Novagen) having His Tag sequence.

First, the extracted DNA was cut with *Nde* I and *Xho* I, and then, ligated into *Nde*I/*Xho*I-restricted pET26B(+) vector (Novagen) using T4 DNA ligase at 160 for 24 hours. The ligated recombinant expressing vector is named as 'pET-hTyrosinase(TM-)'. After that, *E. coli* BL21(DE3) was transformed with the pET-hTyrosinase(TM-) by heat shock (42□). The transformed *E. coli* BL21(DE3) was cultured on an LB-Agar plate including 50µg/mℓ of kanamycin for 12 hours. A colony was selected from each plate and was used for a mass expression of the recombinant human tyrosinase. The recombinant human tyrosinase expressed from the transformed *E. coli* according to the present invention was expressed as a fusion protein in which six molecules of histidine were combined.

### <Example 2>

### Expression and purification of recombinant human tyrosinase protein using pET-hTyrosinase(TM-)

As described in Example 1, the *E. coli* transformed with the expression vector pET-hTyrosinase(TM-) obtained by recombining the expression vector pET26 and Human tyrosinase cDNA, was cultured in a 1L of LB broth including 30µℓ/mℓ of kanamycin, at 37□ for 2 hours. When absorbance became about 0.4-0.5, 0.4mM of IPTG (isopropyl β-D-thoigalactopyranoside) was added to the broth to induce a expression of an enzyme and the broth was further cultured for about 8-10 hours, and then, a centrifugation was conducted at 8,000g for 10 minutes to collect the *E. coli.* A 20mM of tris-Cl buffer solution (pH 7.9; hereinafter, this solution will be called as "buffer solution A") including 500mM of NaCl and not including triton X-100 was added to resuspend the *E. coli.* After that, the resuspended E *coli* was homogenized at 4□ for 20 minutes by using Sonics amp(Materials INC.), while applying periodically a pulse with 30-40 watts, 8% of amplitude for 9 seconds, then stopping for 1 second. A crude extract was obtained by centrifugation at 8,000g for 20 minutes. The crude extract was injected and adsorbed by His-bind resin chromatography(Novagen) and then washed sufficiently using buffer solution A including 50mM of imidazole to remove unnecessary protein. At this time, the washing was continued until OD 280 value of the washed solution was zero (0). Then, proteins was eluted by the buffer solution A including 500mM of imidazole at a rate of 1mℓ/min. In order to remove imidazole included in the eluting solution, the product was dialyzed three times using 20mM tris-Cl buffer solution (pH 7.9) for 8 hours, respectively. The purity of the purified tyrosinase was confirmed by SDS-PAGE

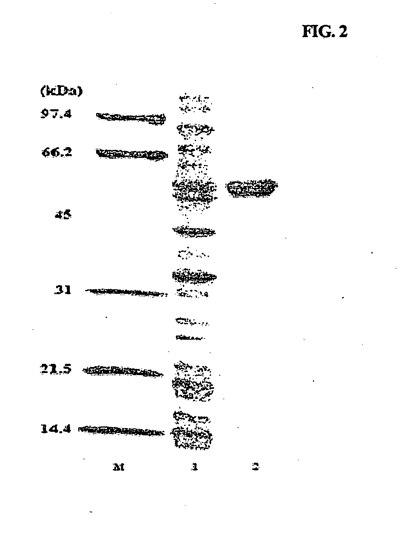

As a result of the above experiment, the recombinant human tyrosinase of the present invention has the molecular weight of about 53 kDa, as shown in FIG. 2. Generally, the tyrosinase of mammal is known as increasing the molecular weight thereof due to glycosylation during post-translational modification (Imokawa and Mishima, J. Invest. Derm. 85: 165-168, 1985; Imokawa, J. Invest. Derm. 95: 39-49, 1990). The molecular weight of the glycosylated tyrosinase is known to about 70 kDa (Laskin and Paccinini, J. Biol. Chem. 261: 16626-16635, 1986). However, it is confirmed that the recombinant human tyrosinase of the present invention expressed in E. coli according to the present invention is not glycosylated from the result shown in Fro. 2.

### <Comparative example 1 >

Preparation of human tyrosinase according to a method disclosed in Korean Patent Application No. 2001-0057379

Human tyrosinase was produced according to a previous method disclosed in Korean Patent Application No. 2001-0057379 conducted by the present inventors.

*E. coli* transformed with expression vector pHis-hTyr obtained by recombining expression vector pTrcHis and human tyrosinase cDNA was cultured in 1L of LB Broth including 50µℓ/mℓ of ampicillin, at 37□ for 2 hours. When absorbance at 600nm became about 0.4-0.5, 1mM of IPTG (isopropyl β-D-thoigalactopyranoside) was added to induce the expression of an enzyme. After culturing for about 8-10 hours, centrifugation was conducted at 8,000g for 10 minutes to collect *E. coli.* Then, 20mM potassium phosphate buffer solution/1% triton X 100 having pH 6.8 (which will be called buffer solution B, hereinafter) was added to resuspend the *E. coli.* Then, the resuspended *E*. *coli* was homogenized using a Sonics amp. The homogenate was centrifugated at 20,000g and 40 for 10 minutes. The supernatant was injected into DEAE-Sephacel column equilibrated using the buffer solution B. Polyhistidine-tyrosinase fusion protein, which had not combined to the column but eluted, was injected into a immobilized metal affinity column equilibrated using 50mM potassium phosphate buffer solution/50mM NaCl/1% triton X 100 (pH 7.8, which will be called as buffer solution C, hereinafter). The column was washed using the buffer solution C several times. Then, the fusion protein was eluted using the buffer solution C including 50mM of imidazole. Then, the fusion protein was dialyzed with 50mM tris-HCl/5mM CaCl₂ (pH 8.0, which will be called as buffer solution D, hereinafter). The fused peptide was cut by reacting in the buffer solution D having an excessive amount of enterokinase (Biozyme)(enzyme:substrate = 1:50) at 370 for 2 hours, and thus cut fused peptide (polyhistidine peptide) was removed by injecting into a immobilized metal affinity column. Non-combined enzyme portion was dialyzed with the buffer solution B to obtain purified recombinant tyrosinase.

### <Comparative example 2>

Preparation of human tyrosinase according to a method disclosed in Korean Patent Application No. 2002-0073728

Human tyrosinase was produced according to the previous method disclosed in Korean Patent Application No. 2002-0073728 by the present inventors.

Transformed *E*. *coli* BL21 (DE3) with pET-hTyr expression vector was cultured and collected. The *E*. *coli* washed 2-3 times using a 20mM potassium phosphate buffer solution including 1% triton X-100 (pH 6.8, which will be called buffer solution E, hereinafter) and resuspended using 10mℓ of the buffer solution E. After that, the *E*. *coli* was homogenized using a Sonics amp. (Materials INC.). The homogenate was centrifugated at 20,000g and at 4□ for 20 minutes to obtain crude extract. Supernatant was separated and then was adsorbed onto DEAE-Sephacel ion exchange chromatography (Pharmacia Biotech, Uppsala, Sweden) equilibrated using the buffer solution E. The column was eluted by the rate of 1mℓ/min. The eluted solution was collected and was dialyzed three times using 20mM tris-HCl buffer solution including 1 % triton X-100 and 500mM of NaCl (pH 7.9, which will be called buffer solution F, hereinafter) for 8 hours, respectively. This process was repeated for three times. The dialyzed solution was adsorbed onto His-bind resin chromatography (Novagen) equilibrated using the buffer solution F. After that, the column was sufficiently washed using the buffer solution F including 10mM of imidazole to remove unnecessary protein. At this time, the washing was continued until the OD 280 value of the washed solution was zero (0). Then, the protein was eluted again using the buffer solution F including 100mM imidazole at a rate of 1mℓ/min. The eluted solution was dialyzed three times using the buffer solution E for 8 hours, respectively. The dialyzed solution was centrifugated at 20,000g and at 4□ for 20 minutes to separate recombinant human tyrosinase.

### <Test Example 1>

### Comparison of tyrosine hydroxylase activity of human tyrosinase

Tyrosine hydroxylase activity of the recombinant human tyrosinase prepared in example 2 and in Comparative example 2 were compared.

The measuring of the tyrosine hydroxylase activity was conducted according to a method of Husain et al. (Husain et al., J. Invest. Dermatol. 78: 243-252, 1982). Hydroxylation was conducted within an L-Dopa concentration range of 0-30mM. A standard curve with respect to L-dopa was obtained. Using the standard curve, the amount of L-dopa produced after the reaction was determined. The reaction solution was prepared by adding 100µM of L-tyrosine, L-dopa of each concentration, 4mM of ascorbic acid and 100µℓ of human tyrosinase into 50mM Potassium phosphate buffer solution (pH 6.8) to obtain 2.5mℓ of the solution. After that, the reaction was conducted at 37□ for 3 hours. At this time, a reaction was conducted without adding the purified human tyrosinase as a control. The product was subjected to excitation at 360nm and then was subjected to an emission at 490nm by a method of Ram et al. (Ram et al., J. Biol. Chem. 267: 23707-23712, 1992). The intensity of the emission was measured. The measured value was converted into the concentration of L-dopa and the enzyme activity was measured. The specific activity with respect to the tyrosine hydroxylase activity of the purified human tyrosinase of the present invention was determined as the created mol number (mmol) of the L-dopa with respect to the amount of the enzyme(µg) per hour.

**□Table 1□**

| Activity of tyrosine hydroxylase of recombinant human tyrosinase | | |
|---|---|---|
| | Purification step | Specific activity (µmol/mg/h) |
| Recombinant human Tyrosinase of example 2 (pET-hTyrosinase(TM-) | Crude extract | 2.1±0.05 |
| | His·bind resin chromatography | 108.3±2.10 |
| Recombinant human tyrosinase of comparative example 2(pET-hTyr) | Crude extract | 1.8±0.05 |
| | DEAE-Sephacel chromatography | 11.0±0.12 |
| | His-bind resin chromatography | 88.0 ± 1.50 |

As a result of measurement, for the recombinant human tyrosinase produced in E. *coli* using pET-hTyr expression vector (Korean Patent Application No. 2002-0073728), the specific activity with respect to tyrosine hydroxylase activity of the finally purified recombinant human tyrosinase by means of His-bind resin chromatography was increased by about 49 times when comparing to that of the crude extract, as illustrated in Table 1. However, the hydroxylase activity of the transmembrane removed recombinant tyrosinase according to the present invention was increased by about 52 times than that of the crude extract. Meantime, the specific activity with respect to the tyrosine hydroxylase activity of the purified tyrosinase from human HeLa cell is 22.3±0.01 pmol/µg/h (Spritz et al., J. Invest. Dermato., 109; 207-212, 1997). Accordingly, it is confirmed that the transmembrane removed recombinant human tyrosinase expressed in E. *coli* according to the present invention has superior tyrosine hydroxylase activity.

### < Test Example 2>

### Comparison of dopa oxidase activity of human tyrosinase

The dopa oxidase activity of the recombinant human tyrosinase produced from example 2 was compared with that produced from comparative examples 1 and 2.

The measurement of the dopa oxidase activity was conducted by the method of Fling et al. (Fling et al. J. Biol. Chem. 238: 2045-2053, 1963). The transformation ofL-dopa which was the substrate of dopa oxidase into a brown product of dopachrome by tyrosinase was measured at 475nm. Dopachrome exhibited maximum absorbance with respect to the concentration at 475nm and the mole absorbance coefficient at this point was 3600 (M⁻¹cm⁻¹). 3mM of L-dopa was added into 50mM of tris-HCl buffer solution (pH 7.5) and incubated at 37□ for 10 minutes. Then, 30µℓ of human tyrosinase was added and the absorbance change was measured at 475nm for 3 minutes. At this time, the unit of the activity of the enzyme was defined as follows. When 1µmole of dopachrome was produced for 1 minute, this activity was defined as 1 unit (µmole/min). Specific activity was defined as 1 unit per 1 mg enzyme.

**□Table 2□**

| Dopa oxidase activity of recombinant human tyrosinase | | | | | | |
|---|---|---|---|---|---|---|
| | Purification step | Total activity (units) | Total protein (mg) | Specific activity (units/mg) | Yield (%) | Purification degree (fold) |
| Recombinant human tyrosinase | Crude extract | 6200 | 350 | 17.7 | 100 | 1.0 |
| of example 2 (pET-hTyrosinase(TM-)) | His-bind resin chromatography | 2977 | 3.69 | 807 | 48 | 46 |
| Recombinant human tyrosinase of comparative example 2 (pET-hTyr) | Crude extract | 5700 | 400 | 14.3 | 100 | 1.0 |
| | DEAE-Sephacel chromatography | 4460 | 64 | 69.7 | 78 | 5 |
| | His·bind resin chromatography | 1922 | 2.75 | 699 | 34 | 49 |
| Recombinant human tyrosinase of comparative example 1 (pHis-hTyr) | Crude extract | 136 | 360 | 0.4 | 100 | 1.0 |
| | DEAD-Sephacel chromatography | 64 | 46 | 1.4 | 47 | 3.5 |
| | His-bind resin chromatography | 26 | 1 | 26.0 | 19 | 65.0 |

From the above result, it is confirmed that the protein amount (the protein amount of the crude extract) of the transmembrane removed recombinant human tyrosinase prepared from example 2 according to the present invention is similar to that of the recombinant human tyrosinase prepared by the methods disclosed in Korean Patent Application No. 2001-0057379 (comparative example 1) and Korean Patent Application No. 2002-0073728 (comparative example 2), as shown in Table 2. However, the total activity of the recombinant human tyrosinase according to the present invention is remarkably higher than that of the recombinant human tyrosinase prepared by the methods disclosed in Korean Patent Application No. 2001-0057379 (comparative example 1) and Korean Patent Application No. 2002-0073728 (comparative example 2). The specific activity of the recombinant human tyrosinase according to the present invention is about 31 times higher than that of the recombinant human tyrosinase prepared by the method disclosed in Korean Patent Application No. 2001-0057379 (comparative example 1) and is about 1.2 times higher than that of the recombinant human tyrosinase prepared by the method disclosed in Korean Patent Application No. 2002-0073728 (comparative example 2). From this result, it is confirmed that the recombinant human tyrosinase of the present invention prepared in *E. coli* according to the method of the present invention does not produce insoluble inclusion body, but is expressed as an enzyme having a complete activity. In addition, it also can be confirmed that the amount of the finally purified enzyme is remarkably greater in the present invention.

### < Test Example 3>

### Influence with respect to the concentration of L-tyrosine

The concentration of the substrate, L-tyrosine was given in a range of 0.01-1mM and the influence of the substrate concentration onto the recombinant human tyrosinase prepared by example 2 was evaluated. It is conducted by the same method of measuring the tyrosine hydroxylase activity explained in the experiment 1. The Km value of the recombinant human tyrosinase according to the present invention was calculated by the Lineweaver-Burk Plot(Lineweaver et al., J. Am, Chem. Soc., 56; 658-666, 1934).

As a result, Km value of the L-tyrosine of the recombinant human tyrosinase was calculated as 1.32µM. This value is lower at the most about 130 times of the Km value of 0.17mM of the human tyrosinase produced by the previous research by the present inventor (Korean Patent Application No. 2001-0057379), the Km value of 2.34µM (Korean Patent Application No. 2002-0073728) and the Km value of 0.2mM of the tyrosinase extracted from a melanoma cell (Sangjin et al., Korean Biochem J. 26: 632-637, 1993).

From the result, it is understood that the affinity of the recombinant human tyrosinase of the present invention onto the substrate, L-tyrosine is higher by 100 times or over. In addition, the result means that the recombinant human tyrosinase of the present invention is suitably folded and the access of the substrate to the active site of the human tyrosinase is further advantageous.

### < Test Example 4>

### Influence to the L-dopa concentration

The concentration of L-dopa, which is another substrate of the human tyrosinase was given in a range of0.05-5mM and the influence of the substrate concentration with respect to the recombinant human tyrosinase prepared in example 2 was examined. The dopa oxidase activity is measured by the same method of experiment 2. After that, the Km value of the recombinant human tyrosinase according to the present invention with respect to the L-dopa was calculated according to the same method in experiment 3.

As a result, the Km value for the L-dopa of the recombinant human tyrosinase according to the present invention was calculated as 0.34mM. This value is similar to the Km value of 0.4mM for the L-dopa of the purified tyrosinase from the melanoma cell (Kang et al., Korean Biochem. J., 26: 632-637, 1993). From the result, it is confirmed that the recombinant human tyrosinase of the present invention expressed in *E*. *coli* has the similar binding affinity with that of the tyrosinase of the melanoma cell.

The present application claims the priority of the Korean Patent Application No. 2006-0091308 filed on September 20, 2006.

### Industrial applicability

As described above, in the present invention a recombinant human tyrosinase gene from which a C-terminal transmembrane domain is removed, was expressed and a recombinant human tyrosinase is prepared by separating and purifying the expressed gene product. The method according to the present invention has effects on mass-producing the recombinant human tyrosinase having a high enzyme activity without forming insoluble inclusion body. In addition, the method according to the present invention has effects on simple purifying process and high yield. Therefore, the present invention can be applied to various industrial field including biological and medical field, food industry, cosmetics, chemical industries, and the like.

Although a preferred embodiment of the present invention has been described for illustrative purposes, those skilled in the art will appreciate that various modifications, additions and substitutions are possible, without departing from the scope and spirit of the invention as disclosed in the accompanying claims.

## Claims

1. An isolated human tyrosinase consisting of the amino acid sequence set forth in SEQ ID NO: 2.

2. An isolated polynucleotide encoding the human tyrosinase of claim 1.

3. The polynucleotide according to claim 2, wherein the polynucleotide consists of the nucleotide sequence set forth in SEQ ID NO: 1.

4. A recombinant vector comprising the polynucleotide of claim 2.

5. A transformed bacteria comprising the recombinant vector of claim 4.

6. A method of producing the human tyrosinase of claim 1, comprising culturing the transformed bacteria of claim 5.

7. The method according to claim 6, wherein the method further comprises purifying the cultured product by means of an immobilized metal affinity column chromatography.

## Patentansprüche

1. Isolierte humane Tyrosinase, welche die Aminosäuresequenz wie in SEQ ID NO: 2 dargestellt, aufweist.

2. Isoliertes Polynukleotid, welches für die humane Tyrosinase aus Anspruch 1 kodiert.

3. Polynukleotid nach Anspruch 2, wobei das Polynukleotid die Nukleotidsequenz wie in SEQ ID NO: 1 dargestellt, aufweist.

4. Rekombinanter Vektor umfassend das Polynukleotid aus Anspruch 2.

5. Transformierte Bakterien, welche den rekombinanten Vektor nach Anspruch 4 aufweisen.

6. Verfahren zur Herstellung der humanen Tyrosinase aus Anspruch 1, welches das Kultivieren der transformierten Bakterien aus Anspruch 5 umfasst.

7. Verfahren nach Anspruch 6, wobei das Verfahren weiterhin das Aufreinigen des kultivierten Produktes mit Hilfe einer immobilisierten Metall-Affinitätssäulen-Chromatographie umfasst.

## Revendications

1. Tyrosinase humaine isolée constituée de la séquence d'acides aminés représentée par SEQ ID N° 2.

2. Polynucléotide isolé codant la tyrosinase humaine de la revendication 1.

3. Polynucléotide selon la revendication 2, dans lequel le polynucléotide est constitué de la séquence de nucléotides représentée par SEQ ID N° 1.

4. Vecteur recombiné comprenant le polynucléotide de la revendication 2.

5. Bactérie transformée comprenant le vecteur recombiné de la revendication 4.

6. Procédé de production de la tyrosinase humaine de la revendication 1, consistant à cultiver la bactérie transformée de la revendication 5.

7. Procédé selon la revendication 6, dans lequel le procédé consiste en outre à purifier le produit cultivé au moyen d'une chromatographie sur colonne d'affinité de métal immobilisé.
